## Europäisches Patentamt

## European Patent Office

## Office européen des brevets

(19)

(11) Publication number: **0 001 861**
**B1**

(12) # EUROPEAN PATENT SPECIFICATION

(45) Date of publication of patent specification: **14.10.81**

(21) Application number: **78200272.9**

(22) Date of filing: **30.10.78**

(51) Int. Cl.³: **C 07 C 31/28,**
**C 07 C 53/126, C 07 F 3/02**

(54) **Process for the preparation of hydrocarbon solutions of organic magnesium compounds containing oxygen and the solutions obtained.**

(30) Priority: **31.10.77 NL 7711923**

(43) Date of publication of application:
**16.05.79 Bulletin 79/10**

(45) Publication of the grant of the European patent:
**14.10.81 Bulletin 81/41**

(84) Designated Contracting States:
**BE DE FR GB SE**

(56) References cited:
**DE - A - 1 520 027**
**FR - A - 2 117 223**
**GB - A - 1 272 156**

(73) Proprietor: **STAMICARBON B.V.**
**Postbus 10**
**NL-6160 MC Geleen (NL)**

(72) Inventor: **Van Den Berg, Cornelis Emile Petrus**
**Valerius**
**Guido Gezellelaan 34**
**NL-6165 EK Geleen (NL)**

(74) Representative: **De Boer, Jan et al,**
**OCTROOIBUREAU DSM Postbus 9**
**NL-6160 MA Geleen (NL)**

Courier Press, Leamington Spa, England.

## Process for the preparation of hydrocarbon solutions of organic magnesium compounds containing oxygen and the solutions obtained

The invention relates to a process for the preparation of hydrocarbon solutions of organic magnesium compounds wherein two organic groups are each attached to magnesium by an oxygen atom.

'Organic magnesium compounds containing oxygen' here denotes compounds in which arbitrary organic groups have been attached to magnesium by way of an oxygen atom. It should be noted that metal compounds, especially magnesium compounds, in which an organic group is attached to a metal atom, particularly the magnesium atom, by way of a carbon atom, are called organometallic compounds or organomagnesium compounds and should not be confused with the organic metal compounds meant here. The organic groups attached to magnesium by way of an oxygen atom may be saturated or unsaturated, their chains straight or branched, or they may be cyclic. These groups may be attached to magnesium at each carbon atom of the chain by way of an oxygen atom. These groups may also contain hetero atoms, such as oxygen, sulphur, nitrogen, phosphorus, halogen. By preference, these groups contain no hetero atoms, or only one or more oxygen atoms, i.e. hydrocarbyl groups may have been attached by way of an oxygen atom, such as alkyl or cycloalkyl groups or monounsaturated or polyunsaturated groups derived therefrom, aryl groups, alkaryl or aralkyl groups, or hydrocarbyl groups that may be substituted by one or more hydroxy, oxo (i.e. with one or more terminal or non-terminal carbonyl groups), and/or carboxyl groups. In the present invention the term 'organic magnesium compounds containing oxygen' especially denotes the magnesium alkoxides, magnesium aroxides, i.e. derivatives of phenol or homologues of phenol, magnesium carboxylates, i.e. the magnesium salts of carboxylic acids, both aliphatic and aromatic, and magnesium chelates of 1.3-diketones, in particular the magnesium acetyl acetonate. The organic magnesium compounds containing oxygen are preferably magnesium alkanolates.

Magnesium alkanolates and their preparation have long been known. According to Gmelin's Handbuch der Anorganischen Chemie, edition 8 Magnesium, system No. 27, volume A, pp. 315 and 316, it was already known at the turn of the century that magnesium dissolves in absolute methanol at room temperature to form magnesium methylate. The reaction with ethanol is found to be more difficult, and the reaction with propanol or higher alcohols very difficult, e.g. only in the gaseous phase with heated magnesium, or even no reaction takes place. Methods of preparing and forming magnesium alkanolates have also been described in Beilstein's Handbuch der Organischen Chemie under the entries methanol, ethanol, propanol, isopropanol and higher alcohols, system numbers 19, 20 and 24 in the main volume and the supplementary volumes.

Magnesium methanolate is soluble in methanol, magnesium ethanolate is appreciably less so in ethanol. As the number of carbon atoms in the alkoxy groups increases, the solubility in the corresponding alcohol rapidly decreases further. Indeed, magnesium propylate or isopropylate is virtually insoluble in the corresponding alcohol.

According to United States Patent Specification 2,570,058, magnesium can be converted with alkanols with at least 4 carbon atoms if this conversion is effected in the presence of chloroform or carbon tetrachloride and a catalytic amount of a mercury compound. If the conversion of magnesium with, e.g., n-amyl alcohol is effected in the presence of a small amount of a mercury compound, but in the absence of chloroform or carbon tetrachloride, the reaction soon stops and a deposit can be observed on the surface of the magnesium metal.

However, if the conversion is effected with a mixture of 95% of n-amyl alcohol and as little as 5% of carbon tetrachloride and with a catalytic amount of mercury compound, the reaction proceeds until the magnesium has dissolved completely. The chlorine compound obviously has a solubilizing effect, which can also be observed when a mixture of an alcohol and a hydrocarbon solvent is used. Nothing is stated about the solubility in hydrocarbons. Magnesium aroxides, especially magnesium phenolate, are also well-known compounds. Magnesium phenolate can be prepared in an aqueous medium from phenol and magnesium oxide or magnesium hydroxide.

Of the magnesium chelates of 1,3-diketones, magnesium acetyl acetonate is mentioned in particular, which can be prepared by conversion of acetyl acetonate with magnesium carbonate in water or with magnesium oxide in alcohol. Applicant's GB-PS 1.358.437 (corresponding to FR-PS 2.117.223) states that magnesium alcoholates as such are not soluble in hydrocarbons or can be dissolved only to form very viscous solutions. This specification also mentions some measures to obtain less viscous solutions. It discloses that the magnesium alcoholates can be solubilized, by e.g. aluminium compounds. It is supposed, although this must not be considered a statement binding upon the applicant, that some sort of magnesium polymers are present in the viscous liquids, as described by Bryce-Smith, and Wakefield in J. Chem. Soc. 1964 (July) 2483-5.

The abovementioned patent specification does not describe a straightforward method of dissolving magnesium alkanolates and the methods described give less favourable results in a number of cases.

The GB-PS 1.272.156 discloses new mixed acyl metal alcoholates of the general formula $Me^1Me^2(OR)_x(Ac)_y$. Among the compounds of this kind is $Mg(Ti(C_8H_{17}O)_4(C_{15}H_{31}COO))_2$ that is prepared from magnesium-palmitate and titaniumoctylate and wherein the atomic ratio Ti : Mg = 2 : 1. Said mixed acyl metal alcoholates are used in particular as thickeners in varnishes and lubricating oil.

It has now been found that slightly viscous solutions of organic magnesium compounds containing oxygen can be prepared by dissolving in a hydrocarbon solvent a magnesium compound of this type, in which each of the groups attached to magnesium by way of an oxygen atom contains 3 to 30 carbon atoms, and the two together contain at least 7 carbon atoms, in the presence of 5—100 moles per cent, calculated to the magnesium compound, of an organic compound of a translation metal from groups IV through VI of the Periodic Table of Elements, in which the organic groups each are attached to the transition metal atom by an oxygen atom. Magnesium alkanolates with 3 to 30 carbon atoms per alkoxy group and at least 7 carbon atoms in the two alkoxy groups can be dissolved in a hydrocarbon solvent, preferably with gentle heating, while an organic compound of a transition metal containing oxygen is added. Heating is generally effected at at least 50°C and, preferably, at least 70°C. Solvents with low boiling points, such as propane, butane, pentane make it necessary to effect the process under pressure. If such solvents with low boiling do not deserve special preference, e.g. with a view to the use of the solutions, solvents with higher boiling points will preferably be chosen to enable the dissolving to be effected at atmospheric pressure. The dissolving can advantageously be effected with gentle heating and reflux cooling. When hydrocarbons or hydrocarbon fractions with higher boiling points are used as the solvent, higher temperatures, e.g. of over 100 or even 200°C, may also be used, which is sometimes found to be advantageous and, at any rate, increases the conversion and dissolving rates. For economical reasons heating will not be done at a higher temperature than will be necessary for smooth dissolution. Although the magnesium alkanolates of the lower alcohols can be prepared directly from magnesium and the relative alcohol, the preparation of magnesium alkanolates with alkoxy groups with 5 or more carbon atoms must generally be started from magnesium alkanolates of the lower alcohols, e.g. magnesium methanolate, magnesium ethanolate or magnesium isopropanolate, and then heating them with a higher alcohol, e.g. hexanol, octanol, decanol etc. to convert them into the magnesium alkanolate of the higher alcohol, while the lower alcohol is liberated. A drawback of these conversions is that the removal of the lower alcohol from the reaction mixture is usually highly incomplete, so that the reaction proceeds incompletely. For instance, when magnesium isopropanolate is heated in a hydrocarbon solvent with an amount of decanol that can replace one or both isopropoxy groups, the isopropanol is liberated, which can at first be removed from the reaction mixture by boiling. Before long, however, a very viscous solution of magnesium isopropanolatedecanolate or of magnesium didecanolate in the hydrocarbon solvent will form. Isopropanol can no longer be removed from this very viscous mass by boiling and the conversion cannot be continued to completion. To prepare the magnesium didecanolate, a great excess of decanol may be added, if so desired, and the reaction can be carried on to completion thanks to the strong dilution, but the replacement of the excess decanol by a hydrocarbon solvent, which usually has a lower boiling point than decanol (231°C), is cumbersome and expensive.

The present process is particularly suitable for the direct preparation, from a magnesium alkanolate having 1—3 carbon atoms in the alkoxy groups, of a magnesium alkanolate having a higher number of carbon atoms in the alkoxy groups, by adding a transition-metal compound containing organic oxygen, e.g. a tetra alkoxy titanium compound, during the conversion of a magnesium alkanolate with 1—3 carbon atoms by means of a higher alkanol, e.g. an alkanol with 4—30 carbon atoms.

If magnesium isopropanolate together with e.g. 20 moles % of tetra butoxy titanium, related to the magnesium, and one or two equivalents of decanol, is introduced into an organic solvent, and the mixture is heated, a low-viscosity solution of magnesium isopropanolatedecanolate, or of magnesium didecanolate, is formed, from which the liberated isopropanol can readily be evaporated until full completion of the reaction. The solvent chosen for this conversion is preferably one that can easily be separated from isopropanol by distillation. The solvent used and the final solution must contain, apart from the organic compounds containing oxygen, no or only a small amount of oxygen compounds, e.g. the lower alcohol formed in the conversion of a magnesium alcoholate with a higher alcohol. At any rate the amount of oxygen in such compounds must be smaller than the amount of oxygen by means of which an organic group is attached to the metal. Such compounds are preferably absent, and effective separation of the resulting alcohol and hydrocarbon solvent is therefore desirable.

What was said above by way of example about the conversion of magnesium isopropanolate with decanol naturally also applies to the conversion of any other magnesium alkanolate of an alkanol with at most 3 carbon atoms with a higher alkanol, e.g. an alkanol having 4—30 carbon atoms. Magnesium alcoholates can be prepared directly from butanol or pentanol, it is true, and they can then be converted with higher alcohols in a similar way. But a process of this type is hardly attractive. It is much easier to prepare a methanolate, ethanolate, propanolate or isopropanolate and hence the use of these starting materials is to be preferred for the preparation of a magnesium alcoholate from a higher alkanol. The preparation of magnesium butanolate or pentanolate is easier by way of the magnesium compound of a lower alcohol than by the direct route. Hence, the preparation via a lower alkanolate is also to be preferred in these cases.

By preference, one of the alkoxy groups of the magnesium alcoholates to be dissolved contains at least 5 carbon atoms, for the solubility becomes better as the number of carbon atoms in the alkoxy groups is greater.

Without the transition metal compounds, very viscous solutions, or even solid gels, that are very difficult or impossible to handle or process are obtained at concentrations as low as less than 0.5 Molar, e.g. 0.1 or 0.2 M. In general, solutions of magnesium alcoholates in which different alkoxy groups are attached to the magnesium are less viscous than when the alkoxy groups are the same. Consequently, magnesium alcoholates with different alkoxy groups are to be preferred.

These solutions of miscellaneous alcoholates can also be prepared by heating a mixture of a magnesium alcoholate with a smaller alkoxy group and a magnesium alcoholate with a larger alkoxy group, such as, e.g. magnesium diisopropanolate and magnesium didecanolate. The transition-metal compounds contain, preferably hydrocarbyl groups, which are attached to the transition metal by way of an oxygen atom. These include the alkoxides or alkanolates, the aroxides, particularly the phenolates, the alkane carboxylates, but also the transition-metal chelates of 1.3-diketones, particularly of acetyl acetone. The organic groups attached to a transition-metal atom by way of an oxygen atom contain 1 to 20, preferably 4 to 8, carbon atoms per group. Use is preferably made of the alkanolates of transition metals. Suitable transition-metal compounds are titanium, zirconium, vanadium and chromium compounds, but the titanium compounds are used by preference. Of the titanium compounds, the titanium tetra-butanolate (or tetrabutoxy titanium, abbreviated TBT in most cases) is to be preferred. The slightly viscous solutions according to the invention can be obtained by dissolving a magnesium alkanolate in a hydrocarbon solvent which produces a very viscous solution. Next, a transition metal compound is added, after which the viscosity decreases strongly. Naturally only magnesium alkanolates that are soluble as such in hydrocarbons can be used for this purpose. The magnesium alkanolate is preferably dissolved in the presence of a transition-metal compound.

The amount of transition-metal compound amounts to 5—100 moles % calculated to the magnesium compound. For practical reasons the amount of transition-metal compound will not be chosen too large, and will preferably not be greater than necessary. Over a given concentration of the transition-metal compound, which depends on the conditions, the viscosity of the solution of the magnesium compound will not be reduced further, and, consequently, no greater amounts will generally be used. How much transition-metal compound will give the best results can readily be determined by experiment. The efficacy of the transition-metal compounds depends on the transition-metal and the organic groups. Titanium alcoholates are very effective, but clearly larger amounts of zirconium alcoholates are required. Use is preferably made of at most 60 moles % and, more in particular, at most 35 moles %. The best results, i.e. the least viscous solutions with the smallest amount of transition metal, are obtained with titanium tetra-alcoholates. 5 moles % produce solutions that can well be handled, but may still be slightly viscous. At temperatures below room temperature, especially below 0°C, which may occur during shortage in the open air in moderate climates, solutions of this type can become very viscous. At least 15 moles % of titanium alcoholates will preferably be used then in order to prepare solutions that remain low-viscous even upon prolonged storage at temperatures of, e.g., —10°C.

To promote effectiveness, such titanium tetra alkoxylates will be used in amounts not exceeding 60 moles %, and by preference in amounts not exceeding 35 moles %. Compounds of other transition metals generally require the use of larger amounts.

The hydrocarbons used in the present invention as the solvent are aliphatic or cycloaliphatic hydrocarbons, such as, e.g., propane, butane, isobutane, one or more pentane, hexanes, heptanes etc., cyclopentane, cyclohexane, and homologues thereof, etc. Aromatic hydrocarbons, too, can well be used as solvents, but aliphatic or cycloaliphatic hydrocarbons are to be preferred for practical reasons.

Lower hydrocarbons, such as propane and butane, can be used only under pressure. Use is preferably made of solvents that are liquid at atmospheric pressure. Use may also be made of petroleum fractions that consist of aliphatic and cycloaliphatic hydrocarbons and may contain widely varying amounts of aromatics. By preference, hydrocarbon fractions with too high boiling points are not chosen, as they become ever more viscous with higher molecular weight. The boiling point is below 300°C, preferably below 200°C, and, more in particular, below 120°C, or the upper limit of the boiling range is below said temperatures. The organic magnesium compounds containing oxygen in solution in hydrocarbons, especially the magnesium alcoholates, can be used, for instance, as catalyst components and in organic syntheses.

The invention will be further elucidated by the following examples, without being restricted by them.

### Example 1
Preparation of a solution of $Mg(OC_{10}H_{21})_2$.

48 grams of magnesium cuttings and a pinch of iodine are placed in a 2-litre three-necked flask provided with a stirrer and a reflux condenser. Next, 0·5 litre of absolute methanol is added dropwise.

The reaction proceeds vigorously and a viscous white mass is formed. Excess decanol-1 (0.5 l) is then added and the mixture is stirred at 70°C for three hours. The alcohol is evaporated in vacuo and a grey solid mass is obtained, to which 1.5 litres dry gasoline and 0.2 mole (68 ml) of tetrabutoxy titanium are added. The solid mass dissolved completely when stirred at 70°C. When cooled to room temperature, the solution remains thin.

### Example 2
Preparation of a solution of $Mg(iO\text{-}C_3H_7)$ $(OC_{10}H_{21})$.

250 ml of gasoline, 40 mmoles (4.56 g) of magnesium ethanolate, 40 mmoles (3.1 ml) of isopropanol and 40 mmoles (7.6 ml) of decanol-1 are placed in a 0.5-litre three-necked flask provided with a stirrer, a reflux condenser and gas and liquid inlets. The mixture is kept at 70°C for 48 hours with stirring, while dry nitrogen is passed over the reaction mixture. The gas-vapour flow is discharged through the condenser that is slightly cooled with air and the evaporated gasoline is made up regularly. The insoluble magnesium ethanolate slowly dissolves to form magnesium isopropanolate decanolate. The ethanol liberated is discharged as a vapour together with the nitrogen passed over and the gasoline vapour. A viscous jelly is formed. 4 mmoles (1.36 ml) of tetrabutoxy titanium are added. The viscous jelly gradually becomes less viscous and after 1 hour at 70°C a thin, slightly turbid solution has formed. When no or only 4 moles % of tetrabutoxy titanium is added, a solution of 0.3 mole $Mg(Oi\text{-}C_3H_7)$ $(OC_{10}H_{21})$ in gasoline forms a viscous jelly, of which the viscosity cannot be determined. By addition of 10 moles %, 20 moles% and 30 moles % of tetrabutoxy titanium related to the magnesium compound the viscosity becomes successively 13 0,8 and 0,7 m Pa.s at 25°C.

### Example 3
Preparation of a solution of $Mg(OC_{10}H_{21})$ $(OC_{16}H_{33})$.

8.6 grams (75 mmoles) of magnesium ethanolate in 250 ml of pentamethyl heptane are placed in a 500-ml three-necked flask provided with a stirrer, reflux condenser and gas and liquid inlets. 22.1 ml of cetyl alcohol (hexadecanol-1 $C_{16}H_{33}OH$) are added and heating is effected at 110°C while nitrogen is passed over. After 1 hour 14.3 ml of decanol are added and the solution is stirred at 110°C for 4 hours while nitrogen is passed over continuously.

A viscous mass with jelly-like particles is obtained.

7.5 mmoles (2.6 ml) of tetrabutoxy titanium are then added. The viscous reaction mixture soon becomes less viscous and after one hour the solution is thin and it stays so upon cooling to room temperature.

### Example 4
Preparation of a solution of $Mg(iOC_3H_7)$ $(OC_{10}H_{21})$.

76 mmoles (10.8 g) of magnesium diisopropanolate, 76 mmoles (14.5 ml) of decanol-1 and 250 ml of gasoline are placed in a 0.5 litre three-necked flask provided with a stirrer, a reflux condenser and gas and liquid inlets. The mixture is heated with stirring and kept at 70°C for 4 hours, while a small flow of nitrogen is passed over. The nitrogen laden with isopropanol vapour and gasoline vapour is discharged through the condenser that is cooled slightly with air. The evaporated gasoline is made up at intervals. A viscous solution is obtained. 7.6 mmoles (2.6 ml) of tetrabutoxy titanium are then added to the solution that is kept at 70°C. The solution becomes less viscous and after being stirred at 70°C for 1 hour it is as thin as water and it stays so upon cooling to room temperature.

### Comparative example
Instead of tetrabutoxy titanium, 7.6 mmoles of aluminium triisopropanolate are added to the viscous solution of magnesium isopropanolate decanolate prepared according to example 4. The viscosity of the solution has not changed after 1 hour's stirring at 70°C.

### Example 5
Preparation of a solution of $Mg(iOC_3H_7)$ $(OC_{10}H_{21})$.

72 mmoles (10.2 grams) of magnesium isopropanolate in 250 ml of gasoline and 14.4 mmoles (4.9 ml) of tetrabutoxy titanium are placed in a 0.5-litre three-necked flask provided with a stirrer, a reflux condenser and gas and liquid inlets. This mixture is kept at 70°C for 8 hours with stirring, while nitrogen is passed over. It is found that the magnesium isopropanolate does not dissolve and no change is observed in the system. 72 mmoles (13.7 ml) of n.decanol are then added and the mixture is stirred at 70°C for another 8 hours while nitrogen is passed over. The evaporated gasoline is made up at intervals. A clear solution is formed, which also remains as thin as water upon cooling to room temperature. This solution is still found to be thin after 3 months' storage at −7°C.

Example 6
Preparation of a solution of Mg(iOC$_3$H$_7$) (OC$_8$H$_{17}$).
The process of example 4 is carried out, but 76 mmoles of n-octanol-1 are now added. Here, too, a solution is obtained that is thin at room temperature and the viscosity of which has not changed upon 10 weeks' storage at room temperature.

Example 7
Preparation of a solution of Mg(iOC$_3$H$_7$) (OC$_8$H$_{17}$).
Example 6 is repeated on the understanding that 76 mmoles of octanol-2 are added. A thin solution is obtained which also stays thin upon cooling to room temperature and which distinguishes itself from the solution obtained in example 6 by a slight turbidity only.

Example 8
Preparation of Mg(OC$_{10}$H$_{21}$)$_2$.
76 mmoles (10.8 g) of magnesium diisopropanolate, 152 mmoles (28.9 ml) of decanol-1 and 250 ml of gasoline are placed in a 0.5-litre three-necked flask provided with a stirrer, a reflux condenser and gas and liquid inlets. Next, 7.6 mmoles (2.6 ml) of tetrabutoxy titanium are added. The reaction mixture is heated to 70°C with stirring and kept at this temperature for 4 hours. A small flow of nitrogen is passed over the mixture and the nitrogen laden with isopropanol vapour and gasoline vapour is discharged through the condenser that is slightly cooled with air. The evaporated gasoline is made up at intervals. A thin solution is obtained which stays invariably thin after cooling and 10 weeks' storage at room temperature.

Example 9
Preparation of a solution of Mg(iOC$_3$H$_7$) (OC$_6$H$_{13}$).
A solution is prepared in the same way as in example 4, but 76 mmoles of hexanol-1 are added instead of decanol-1. A very viscous solution is obtained after 1 hour's stirring at 70°C. 7.6 mmoles (2.6 ml) of tetrabutoxy titanium are added and the solution has become as thin as water after 1 hour at 70°C. The solution stays thin upon cooling and storage at room temperature.

Example 10
Preparation of a solution of Mg(iOC$_3$H$_7$) (OC$_5$H$_{11}$).
A solution is prepared in the same way as in example 4, but 76 mmoles of pentanol-1 are added instead of decanol-1. After 1 hour's stirring at 70°C a viscous solution is obtained, the viscosity of which increases further when stirring at 70°C is continued. After 3 hours 7.6 mmoles (2.6 ml) of tetrabutoxy titanium are added. The solution becomes as thin as water after about 10 minutes. The solution is thin at room temperature, but it gradually becomes slightly more viscous upon prolonged storage at room temperature.

Example 11
Preparation of a solution of Mg(iOC$_3$H$_7$) (OC$_{10}$H$_{21}$)
A solution of Mg(iOC$_3$H$_7$) (OC$_{10}$H$_{21}$) is prepared in the same way as in example 4, but 250 ml of cyclohexane are used as the solvent instead of 250 ml of gasoline.
A thin solution is obtained, which also remains thin upon cooling and prolonged storage at room temperature.

Example 12
Preparation of Mg(iOC$_3$H$_7$) (OC$_{10}$H$_{21}$).
Example 4 is repeated, but 7.6 mmoles of tetrabutoxy zirconium are added instead of tetrabutoxy titanium. After 1 hour's stirring at 70°C the viscosity of the solution has decreased appreciably, but the solution has not become thin. Another 7.5 mmoles of tetrabutoxy zirconium are added and a thin solution is now obtained after 1 hour's stirring at 70°C.
After cooling and three day's storage at room temperature the viscosity has clearly increased, although the solution can still be handled well.
Again 7.6 moles of tetrabutoxy zirconium are added (so in all 22.8 mmoles, i.e. 30 moles %) and the solution then obtained is permanently thin.

Example 13
Preparation of a solution of Mg(iOC$_3$H$_7$) (OC$_{10}$H$_{21}$).
A solution is prepared by the process of example 4, but only 3.8 mmoles of tetrabutoxy titanium are added. The viscous solution of magnesium isopropylate decanolate is thin after 1 hour's stirring at 70°C, but this solution turns viscous upon cooling to room temperature.

Example 14
Preparation of a solution of Mg(C$_{17}$H$_{35}$COO)$_2$.
24.3 grams (75 mmoles) of magnesium stearate and 250 ml of pentamethylheptane are placed in a 0.5-litre three-necked bottle provided with a stirrer and a reflux condenser. The mixture is heated at 150°C with stirring, when the magnesium stearate dissolves in the pentamethyl heptane to form a clear viscous solution. 2.6 ml (7.5 mmoles) of tetrabutoxy titanium are now added. The viscosity of the jelly becomes considerably less. Cooling to room temperature again gives rise to a viscous jelly. The jelly is heated again to 150°C with stirring and another 2.6 ml (7.5 mmoles) of tetrabutoxy titanium are added. The viscous solution becomes thin and stays so upon

cooling to room temperature. The solution is allowed to stand at room temperature for 7 days. The viscosity is found to increase, but the solution can still be handled very well.

## Claims

1. Process for the preparation of a solution of a magnesium compound in which two organic groups are attached to a magnesium atom by an oxygen atom, characterized in that a magnesium compound with organic groups which each contain 3—30 carbon atoms and in total contain at least 7 carbon atoms and which are each attached to the magnesium atom by an oxygen atom, is dissolved in a hydrocarbon solvent in the presence of 5—100 moles %, calculated to the magnesium compound, of an organic compound of a transition metal of groups IV to VI of the Periodic Table of Elements in which the organic groups are each attached to the transition metal atoms by an oxygen atom.

2. Process according to claim 1, characterized in that the said magnesium compound is a magnesium dialkanolate.

3. Process according to claim 2, characterized in that the said magnesium dialkanolate contains two different alkoxy groups.

4. Process according to claims 1—3 characterized in that the dissolution is effected by heating.

5. Process according to claims 1—4, characterized in that a solution of a magnesium dialkanolate with 3—30 carbon atoms in each alkoxy group and in total containing at least 7 carbon atoms is prepared by converting a magnesium alkanolate with 1—3 carbon atoms per alkoxy group with 1 to 2 equivalents of an alkanol with 3—30 carbon atoms, in the presence of 5—100 moles % — related to the magnesium compound — of the organic compound of a transition metal.

6. Process according to claims 2—5, characterized in that the said magnesium dialkanolate contains at least one alkoxy group containing at least 5 carbon atoms.

7. Process according to claims 1—6, characterized in that the said transition metal compound is a titanium alkanolate.

8. Process according to claim 7, characterized in that the said titanium compound is tetrabutoxy titanium.

9. Process according to claims 1—8, characterized in that at least 15 moles % of the said transition-metal compound, calculated on the magnesium, are used.

10. Process according to claims 1—9, characterized in that at most 60 moles % of transition-metal compound, calculated to magnesium, are used.

## Patentansprüche

1. Verfahren zur Herstellung einer Lösung einer Magnesiumverbindung, in der zwei organische Gruppen durch ein Sauerstoffatom mit einem Magnesiumatom verbunden sind, dadurch gekennzeichnet, dass eine Magnesiumverbindung mit organischen Gruppen, von denen jede 3—30 Kohlenstoffatome enthält und die insgesamt mindestens 7 Kohlenstoffatome enthalten und von denen jede durch eine Sauerstoffatom an das Magnesiumatom gebunden ist, in einem Kohlenwasserstoff-Lösungsmittel in Anwesenheit von 5—100 Mol %, bezogen auf die Magnesiumverbindung, einer organischen Verbindung eines Übergangsmetalls der Gruppen IV bis VI des Periodensystems der Elemente gelöst wird, in dem die organischen Gruppen jeweils durch ein Sauerstoffatom an die Übergangsmetallatome gebunden sind.

2. Verfahren gemäss Patentanspruch 1, dadurch gekennzeichnet, dass die genannte Magnesiumverbindung ein Magnesiumdialkanolat ist.

3. Verfahren gemäss Patentanspruch 2, dadurch gekennzeichnet, dass das genannte Magnesiumdialkanolat zwei verschiedene Alkoxygruppen enthält.

4. Verfahren gemäss den Patentansprüchen 1—3, dadurch gekennzeichnet, das die Auflösung durch Erhitzen erfolgt.

5. Verfahren gemäss den Patentansprüchen 1—4, dadurch gekennzeichnet, dass eine Lösung eines Magnesiumdialkanolats mit 3—30 Kohlenstoffatomen in jeder Alkoxygruppe und insgesamt mindestens 7 Kohlenstoffatomen durch Umwandlung eines Magnesiumalkanolats mit 1—3 Kohlenstoffatomen je Alkoxygruppe mit 1 bis 2 Aquivalenten eines Alkanols mit 3—30 Kohlenstoffatomen in Anwesenheit von 5—100 Mol % — bezogen auf die Magnesiumverbindung — einer organischen Verbindung eines Übergangsmetalls hergestellt wird.

6. Verfahren gemäss den Patentansprüchen 2—5, dadurch gekennzeichnet, dass mindestens eine Alkoxygruppe des Magnesiumalkanolats mindestens 5 Kohlenstoffatome enthält.

7. Verfahren gemäss den Patentansprüchen 1—6, dadurch gekennzeichnet, dass die erwähnte Übergangsmetallverbindung ein Titanalkanolat ist.

8. Verfahren gemäss Patentanspurch 7, dadurch gekennzeichnet, dass die erwähnte Titanverbindung Tetrabutoxytitan ist.

9. Verfahren gemäss den Patentansprüchen 1—8, dadurch gekennzeichnet, dass — bezogen auf das Magnesium — mindestens 15 Mol % der erwähnten Übergasmetallverbindung verwendet werden.

10. Verfahren gemäss den Patentansprüchen 1—9, dadurch gekennzeichnet, dass — bezogen auf das Magnesium — höchstens 60 Mol % der Übergangsmetallverbindung verwendet werden.

## Revendications

1. Procédé pour la préparation d'une solution d'un composé du magnésium dans lequel deux groupes organiques sont reliés à un atome de magnésium par un atome d'oxygène, caractérisé en ce qu'un composé du magnésium dans lequel des groupes organiques contenant chacun de 3 à 30 atomes de carbone et contenant au total au moins 7 atomes de carbone et qui sont reliés chacun à l'atome de magnésium par un atome d'oxygène, est dissous dans un solvant hydrocarboné en présence de 5 à 100 moles %, par rapport au composé du magnésium, d'un composé organique d'un métal de transition des groupes IV à VI du tableau périodique des éléments dans lequel les groupes organiques sont reliés chacun aux atomes du métal de transition par un atome d'oxygène.

2. Procédé selon la revendication 1, caractérisé en ce que le composé du magnésium est un dialcanolate de magnésium.

3. Procédé selon la revendication 2, caractérisé en ce que le dialcanolate de magnésium contient deux groupes alcoxy différents.

4. Procédé selon l'une des revendications 1 à 3, caractérisé en ce que la dissolution est effectuée par chauffage.

5. Procédé selon l'une des revendications 1 à 4, caractérisé en ce qu'une solution d'un dialcanolate de magnésium ayant de 3 à 30 atomes de carbone dans chaque groupe alcoxy et contenant au total au moins 7 atomes de carbone est préparée en transformant un alcanolate de magnésium ayanet de 1 à 3 atomes de carbone par groupe alcoxy avec 1 à 2 équivalents d'un alcanol ayant de 3 à 30 atomes de carbone, en présence de 5 à 100 moles %, par rapport au composé du magnésium, du composé organique d'un métal de transition.

6. Procédé selon l'une des revendications 2 à 5, caractérisé en ce que le dialcanolate de magnésium contient au moins un groupe alcoxy contenant au moins 5 atomes de carbone.

7. Procédé selon l'une des revendications 1 à 6, caractérisé en ce que le composé du métal de transition est un alcanolate de titane.

8. Procédé selon la revendication 7, caractérisé en ce que le composé du titane est le tétrabutoxy titane.

9. Procédé selon l'une des revendications 1 à 8, caractérisé en ce qu'on utilise au moins 15 moles % du composé du métal de transition, en calculant par rapport au magnésium.

10. Procédé selon l'une des revendications 1 à 9, caractérisé en ce qu'on utilise au maximum 60 moles % du composé du métal de transition, en calculant par rapport au magnésium.